# EUROPÄISCHE PATENTANMELDUNG

(11) **EP 1 891 959 A1**
(43) Veröffentlichungstag der Anmeldung: **27.02.2008**
(21) Anmeldenummer: 06016950.5
(22) Anmeldetag: 14.08.2006
(51) Int. Cl.: A61K 31/57, A61K 31/519, A61P 15/00

(54) **Pharmazeutische Zusammensetzung zur Kontrazeption und zur Verminderung des Risikos angeborener Fehlbildungen**

(71) Anmelder: Bayer Schering Pharma Aktiengesellschaft, 13353 Berlin (DE)
(72) Erfinder: Claussen, Claus, 07743 Jena (DE)
(74) Vertreter: Cramer, Eva-Maria

(57) **Zusammenfassung**

Eine pharmazeutische Zusammensetzung zur Kontrazeption enthält in einer täglichen Dosis - 2.0 mg oder 1.5 mg 17a-Cyanomethyl-17-β-hydroxyestra-4,9-dien-3on (Dienogest) und 0.415 mg 17α-Ethinylestradiol (Ethinylestradiol) und (6S)-5-Methyltetrahydrofolat, vorzugsweise als Kalziumsalz der (6S)-5-Methyltetrahydrofolsäure (Metafolin) gemeinsam mit einem oder mehreren pharmazeutisch annehmbaren Hilfsstoffen/Trägern.

Die erfindungsgemäße pharmazeutische Zusammensetzung realisiert sowohl eine orale Kontrazeption als auch ein Mittel zur Verminderung des Risikos angeborener Fehlbildungen.

Ferner enthält ein Kit 21 tägliche Dosiseinheiten der pharmazeutischen Zusammensetzung und 7 tägliche Dosiseinheiten enthaltend allein (6S)-5-Methyltetrahydrofolat, auch vorzugsweise Metafolin.

## Beschreibung

### Technisches Gebiet

Die Erfindung betrifft eine pharmazeutische Zusammensetzung zur Kontrazeption und zur Verminderung des Risikos angeborener Fehlbildungen, welche in einer täglichen Dosis
- 2.0 mg 17α-Cyanomethyl-17-β-hydroxyestra-4,9-dien-3on (Dienogest) und 0.015 mg 17α-Ethinylestradiol (Ethinylestradiol) und (6S)-5-Methyltetrahydrofolat ((6S)-5-MTHF) oder
- 1.5 mg Dienogest und 0.015 mg Ethinylestradiol und (6S)-5-Methyltetrahydrofolat ((6S)-5-MTHF) gemeinsam mit einem oder mehreren pharmazeutisch annehmbaren Hilfsstoffen/Trägern enthält.
Ferner betrifft die Erfindung ein Kit, welches 21 tägliche Dosiseinheiten der vorbezeichneten Wirkstoffkombination und 7 tägliche Dosiseinheiten mit (6S)-5-MTHF enthält.
Auch betrifft die Erfindung eine Tablette, vorzugsweise eine Filmtablette, mit vorbezeichneter Wirkstoffkombination. Im Tablettenkern ist Dienogest anteilig, (6S)-5-MTHF nicht oder anteilig oder als Gesamtgehalt enthalten und im Filmüberzug ist der andere Teil an Dienogest, (6S)-5-MTHF nicht oder anteilig oder als Gesamtgehalt und Ethinylestradiol als Gesamtgehalt enthalten.

### Stand der Technik

Orale empfängnisverhütende Mittel, bestehend aus einer Gestagenkomponente und einer Östrogenkomponente kamen erstmals in den frühen 60er Jahren auf den Markt. Drei wesentliche Eigenschaften charakterisieren das Profil der "Verhütungspille": kontrazeptive Sicherheit, eine sehr gute Zykluskontrolle sowie ein Minimum an Nebenwirkungen.
Seit Einführung hormoneller Kontrazeptiva ist die Forschung auf die Möglichkeit zur Schaffung von Arzneiformen gerichtet, die bei gleichbleibend guter kontrazeptiver Sicherheit und Zykluskontrolle unerwünschte Nebenwirkungen, wie beispielsweise arterielle und venöse Thrombosen und Beeinflussung des Kohlehydrat- und Fettstoffwechsels - hervorgerufen durch einen höheren Ge halt an Gestagen und Estrogen als zur Empfängnisverhütung notwendig - , reduziert.
WO 98/004269 offenbart u. a. die orale Verabreichung einer Kombination von 250 µg - 4 mg Dienogest und 10 µg - 20 µg Ethinylestradiol zur Empfängnisverhütung. Um die wesentliche Verringerung des pro Zyklus verabreichten gesamten empfängnisverhütenden Steroids zu erreichen, während eine gute Zykluskontrolle beibehalten wird, verabreicht man die niedrig dosierte Gestagen/Östrogen-Kombination für 23 bis 25 Tage eines 28-tägigen Menstruationszyklus. Jedoch werden in der Patentschrift keine Ergebnisse und Angaben offenbart, die belegen, dass die erfinderische Idee auch zum Erfolg führt und welche Art der Freisetzung der Steroide angestrebt wird.
Folsäure, auch Pteroyl-mono-glutaminsäure, N-(4-(((2-Amino-1,4-dihydro-4-oxo-6-pteridinyl)methyl)-amino)benzoyl)glutaminsäure (Summenformel: C₁₉H₁₉N₇)O₆), Folinsäure genannt, ist ein hitze- und lichtempfindliches, wasserlösliches Vitamin aus dem Vitamin-B-Komplex (Vitamin B₉).
Es ist bekannt, dass in der Nahrung Folate überwiegend als Pteroylpolyglutamate vorliegen. Diese werden nach Nahrungsaufnahme zunächst in den Mukosazellen zu Pteroylmonoglutamaten hydrolisiert, dann im Darm durch aktiven Transport hauptsächlich resorbiert.
In der Leber werden die überwiegend nicht-methylierten Folate in methylierte Folate umgewandelt und hauptsächlich als 5-Methyltetrahydrofolat (5-MTHF) an Albumin Und α-Makroglobulin gebunden an die Zellen weiter-transportiert, dort aufgenommen, demethyliert und in die Polyglutamatform umgewandelt.
An der Demethylierung sind die Aminosäure Homocystein sowie ein Enzym, welches Vitamin B₁₂ als Coenzym benötigt, beteiligt.
Ferner ist bekannt, dass Verluste am Folatgehalt von Lebensmitteln durch die Zubereitung (Kochen) sowie die Lagerung entstehen können. Weiterhin ist bekannt, dass auf die menschliche Haut treffende intensive UV-Strahlung die Folsäure im Körper reduziert. Hellhäutige Menschen sind dabei besonders betroffen.
Bei unzureichender Versorgung mit Folat und/oder Vitamin B₁₂ wird der Homocysteinstoffwechsel behindert, als Folge kann die Konzentration von Homocystein im Blut ansteigen. Die Konzentration an Homocystein im Blut kann demnach als ein Indikator für den Folatgehalt herangezogen werden.

Der Zustand der Hyperhomocysteinämie wird nach Malinow, MR et al, Homocyst(e)ine, diet, and cardiovascular disease, Statement for healthcare professionals from the Nutrition Committee, American Heart Association. Circulation 99, 178-182, 1999, durch folgende Konzentration im Plasma definiert: 16 - 30µmol/l (moderat); 31 - 100µmol/l (mittel); > 104µmol/l (schwer). Eine Konzentration Ober 10µmol/l wird als kritisch angesehen und ab 12µmol/l besteht Handlungsbedarf.
Neben dem Mangel an Folat und dem Vitamin B₁₂ können aber auch Enzymdefekte den Anstieg der Homocysteinkonzentration bewirken. Der Zusammenhang zwischen erhöhten Homocysteinkonzentrationen im Blut und Gefäßerkrankungen, beispielsweise auch als Risikofaktor für Herz-Kreislauf-Erkrankungen wird seit einiger Zeit diskutiert.
Ebenso wird diskutiert, ob Folsäure/Folat wegen seiner Bedeutung für die DNA-Methylierung und die DNA-Strangstabilität vor malignen Erkrankungen schützen kann.
Auch ist bekannt, dass ein unzureichender Folatstatus in der Schwangerschaft zu angeborenen Fehlbildungen, wie beispielsweise angeborene Herzfehler, angeborene Fehlbildungen der Harnwege, eine akute lymphoblastische Leukämie, Lippen-, Kiefer- und Gaumenspalten oder Fehlbildungen des Zentralnervensystems, wie Neuralrohrdeffekte (Spina bifida oder A-nencephalie) führen können.
Die Deutsche Gesellschaft für Ernährung e.V. empfiehlt deshalb als Tagesdosis grundsätzlich 400 µg Folsäure, für Schwangere 600 µg und für stillende Mütter 600 µg. Dies ist eine globale Aussage. Mangel an Vitamin B₁₂ und Folatmangel weisen im Blutbild identische Veränderungen auf. Durch Verabreichung von Folat/Folsäure kann der Folatmangel kompensiert werden, jedoch der Mangel an Vitamin B₁₂ wird nicht angezeigt. Es ist damit die Gefahr eines maskierten Vitamin-B₁₂-Mangels gegeben.
Die Patentschrift US 6,190,693 B1 offenbart eine Methode der Verabreichung von Folsäure gleichzeitig mit einem konventionellen oralen Kontrazeptivum zur Verwendung als orales Kontrazeptivum. Die Veröffentlichungsschrift WO 2003/070255 offenbart ein orales Kontrazeptivum, bzw. ein Kit zur oralen, hormonellen Kontrazeption, welches Estrogene und/oder Gestagene, Tetrahydrofolate und zwingend Vitamin B₁₂ oder optional Vitamin B₆ enthält.

WO 2005/115349 offenbart eine Darreichungsform zur hormonalen Kontrazeption mit hormonhaltigen Tages-Einheiten und hormonfreien Tages-Einheiten, wobei die hormonhaltigen Tages-Einheiten bis höchstens 200µ Folsäure und die hormonfreien Tages-Einheiten größer 200µg Folsäure enthalten.
Die Patentschrift EP 0 898 965 beansprucht die Verwendung von 5-Methyl-(6S)-tetrahydrofolsäure oder deren pharmazeutisch verträgliche Salze zur Vorbeugung von Neuralrohrdefekten.
Die Patentschrift EP 1 044 975 offenbart kristalline Salze der 5-Methyl-(6R,S)-, -(6S)-und-(6R)-tetrahydrofolsäure und der Verwendung als Bestandteil Nahrungsmittelergänzungsstoff.

### Darstellung der Erfindung

Der Erfindung liegt die Aufgabe zugrunde, eine pharmazeutische Zusammensetzung auf Basis von Dienogest und Ethinylestradiol aufzuzeigen, deren steroidale Dosierung reduziert ist und welche gleichzeitig nach Eintritt einer Gravidität das Risiko angeborener Fehlbildungen reduziert.
Diese Aufgabe wird erfindungsgemäß durch eine pharmazeutische Zusammensetzung zur Kontrazeption und zur Verminderung des Risikos angeborener Fehlbildungen, enthaltend in einer täglichen Dosis von 2.0 mg. 17α-Cyanomethyl-17-β-hydroxyestra-4,9-dien-3on (Dienogest) und 0.015 mg 17α-Ethinylestradiol (Ethinylestradiol) und (6S)-5-Methyltetrahydrofolat ((6S)-5-MTHF)
oder 1.5 mg Dienogest und 0.015 mg Ethinylestradiol und (6S)-5-Methyltetrahydrofolat ((6S)-5-MTHF) gemeinsam mit einem oder mehreren pharmazeutisch annehmbaren Hilfsstoffen/Trägern.
(6S)-5-MTHF oder (6S)-5-Methyltetrahydrofolsäure können auch als 5-Methyl-(6S)-tetrahydrofolate oder 5-Methyl-(6S)-tetrahydrofolsäure bezeichnet werden.

In der erfindungsgemäßen pharmazeutischen Zusammensetzung sind als (6S)-5-MTHF die freie Säureform sowie pharmazeutisch verträgliche Salze und Modifikationen der (6S)-5-Methyltetrahydrofolsäure (N-[4-[[(2-amino-1,4,5,6,7,8-hexahydro-4-oxo-5-methyl-(6S)-pteridinyl)methyl]amino]benzoyl]-L-glutaminsäu-re) bezeichnet. Pharmazeutisch verträgliche Salze sollten sowohl pharmakologisch wie auch pharmazeutisch verträglich sein. Solche pharmakologisch und pharmazeutisch verträgliche Salze können Alkali- oder Erdalkalimetall-Salze sein, vorzugsweise Natrium-, Kalium-, Magnesium oder Kalzium-Salze. Vorteilhafterweise ist erfindungsgemäß als (65)-5-MTHF das kristalline Kalziumsalz der (6S)-5-Methyltetrahydrofolsäure (Metafolin) eingesetzt.

Erfindungsgemäß wird das Kalziumsalzes der (6S)-5-Methyltetrahydrofolsäure (Metafolin) in einer Dosierung von 0.4 bis 1 mg, bevorzugt 451 µg, verwendet.

Alternativ kann für (6S)-5-MTHF auch (6R)-5-Methyltetra-hydrofolat in etwa doppelter Dosierung eingesetzt sein.

Auch wird die Aufgabe erfindungsgemäß mit einer Tablette gelöst, vorzugsweise eine Filmtablette, mit vorbezeichneter Wirkstoffkombination. Im Tablettenkern ist Dienogest anteilig, (6S)-5-MTHF nicht oder anteilig oder als Gesamtgehalt enthalten und im Filmüberzug ist der andere Teil an Dienogest, (6S)-5-MTHF nicht oder anteilig oder als Gesamtgehalt und Ethinylestradiol als Gesamtgehalt enthalten, d.h. die Filmtablette besitzt einen Tablettenkern mit einem retardierend freizusetzenden anteiligen Gehalt zum Gesamtgehalt an Dienogest und einem Filmüberzug mit einem nicht retardierend (schnell) freizusetzenden anteiligen Gehalt zum Gesamtgehalt an Dienogest und einem nicht retardierend (schnell) freizusetzenden Gesamtgehalt an Ethinylestradiol - und einem retardierend freizusetzenden anteiligen Gehalt zum Gesamtgehalt an (6S)-5-MTHF im Tablettenkern und einem nicht retardierend (schnell) freizusetzenden anteiligen Gehalt zum Gesamtgehalt an (6S)-5-MTHF im Filmüberzug oder einem retardierend freizusetzenden Gesamtgehalt an (6S)-5-MTHF im Tablettenkern - oder einem nicht retardierend (schnell) freizusetzenden Gesamtgehalt an (6S)-5-MTHF im Filmüberzug.

Der Anteil an Dienogest und der Anteil an (6S)-5-MTHF werden mindestens zu 10 %, vorzugsweise zu 30 % oder gegebenenfalls der Gesamtgehalt an (6S)-5-MTHF nahezu vollständig retardiert nach größer 30 Minuten aus dem Tablettenkern heraus gelöst , wie mit dem Dissolutionstest unter Verwendung von Wasser mit 37 °C als Dissolutionmedium und 50 U/Min als Rührgeschwindigkeit bestimmt.

Die Bestimmung erfolgt nach Ph.Eur. mittels Drehkörbchenapparatur unter Verwendung von 1000 ml Wasser.
Der Anteil an Dienogest ebenso wie der Gesamtgehalt an Ethinylestradiol ebenso wie der Gesamtgehalt an (6S)-5-MTHF aus dem Filmüberzug werden zu mindestens 75 % in maximal 45 Min, vorzugsweise zu 70 % in 30 Min heraus gelöst , wie mit dem Dissolutionstest unter Verwendung von Wasser mit 37 °C als Dissolutionmedium und 50 U/Min als Rührgeschwindigkeit bestimmt.
Es ist auch denkbar, dass in der zweiten Phase gemeinsam mit dem Anteil an Dienogest ein Anteil an Ethinylestradiol vom Gesamtgehalt an Ethinylestradiol verzögert, vorzugsweise aus dem Tablettenkern, freigesetzt wird. Erfindungsgemäß wird die Aufgabe auch durch ein Kit gelöst, welches 21 tägliche Dosiseinheiten von 2.0 oder 1.5 mg Dienogest und jeweils 0.015 mg 17α-Ethinylestradiol und (6S)-5-MTHF, vorzugsweise 451 µg des Kalziumsalzes der 5-Methyl-(6S)-tetrahydrofolsäure (Metafolin)in jeder täglichen Dosiseinheit oder und 7 tägliche Dosiseinheiten mit (6S)-5-MTHF allein ohne Steroidkombination, vorzugsweise 451 µg Metafolin in jeder täglichen Dosiseinheit enthält.
Alternativ kann die Aufgabe auch durch ein Kit gelöst werden, welches 22 bis 24 tägliche Dosiseinheiten von 2.0 oder 1.5 mg Dienogest und jeweils 0.015 mg 17α-Ethinylestradiol und (6S)-5-MTHF und 4 bis 6 tägliche Dosiseinheiten mit (6S)-5-MTHF allein ohne Steroidkombination, vorzugsweise als (6S)-5-MTHF 451 µg des Kalziumsalzes der 5-Methyl-(6S)-tetrahydrofolsäure (Metafolin)in jeder täglichen Dosiseinheit enthält oder - welches 21 bis 24 tägliche Dosiseinheiten der vorbezeichneten Wirkstoffkombination und 4 bis 7 tägliche Dosiseinheiten an Placebo oder hormonfrei oder
- welches 21 bis 24 tägliche Dosiseinheiten von 2.0 oder 1.5 mg Dienogest und jeweils 0.015 mg 17α-Ethinylestradiol und 4 bis 7 tägliche Dosiseinheiten mit (6S)-5-MTHF allein ohne Steroidkombination, vorzugsweise 451 µg des Kalziumsalzes der (6S)- 5-Methyl-(6S)-tetrahydrofolsäure (Metafolin)in jeder täglichen Dosiseinheit enthält.
Auch wird die Aufgabe erfindungsgemäß durch die Verwendung der aufgezeigten Wirkstoffkombination von 2.0 mg oder 1.5 mg Dienogest und jeweils 0.015 mg Ethinylestradiol und (6S)-5-MTHF gemeinsam mit einem oder mehreren pharmazeutisch annehmbaren Hilfsstoffen/Trägern zur Herstellung ei ner pharmazeutischen Zusammensetzung zur Verminderung des Risikos folatmangelbedingter angeborener Fehlbildungen in der Gravidität gelöst. Es kann sich dabei als vorteilhaft erweisen, wenn die Einnahme von (6S)-5-MTHF , vorzugsweise 451 µg des Kalziumsalzes der (6S)-5-Methyl-tetrahydrofolsäure (Metafolin) in jeder täglichen Dosiseinheit gegebenenfalls bis zum Eintritt der Gravidität, während der Gravidität und während der Stillzeit (hier gegebenenfalls auch in höherer Dosierung) erfolgt.

### Ausführungsbeispiele

### Beispiel 1

Es werden Tabletten mit folgender Zusammensetzung hergestellt:
Kern:

| | | |
|---|---|---|
| Dienogest | 2.000 mg | oder 1.500 mg |
| Ethinylestradiol | 0.015 mg | |
| Metafolin | 0.451 mg | |
| Laktose-Monohydrat | 28.720 mg | |
| Maisstärke | 15.000 mg | |
| Maltodextrin | 3.750 mg | |
| Magnesiumstearat | 0.500 mg | |

Als Ethinylestradiol kann auch ein Ethinylestradiol-beta-Cyclodextrin-Komplex eingesetzt werden. Im Falle der Verwendung des Ethinylestradiol-beta-Cyclodextrin-Komplex (1:2) ist maximal bzw. in etwa die zehnfache Menge einzusetzen.
Alle Substanzen werden in geeigneter Weise gemischt und granuliert. Es erfolgt das Aufziehen des Metafolin nach Abschluss des Granulationsprozesses, erneutes Mischen, Tablettierung und gegebenenfalls Befilmung

### Beispiel 2:

Gesunden jungen Frauen im gebärfähigen Alter wird im Abstand von 8 Wochen Blut abgenommen und der Erythrozytenfolatspiegel mit einer validierten mikrobiologischen, immunologischen oder instrumentellen (z. B. HPLC, LC-MS/MS) Methode oder einer geeigneten Kombination dieser Methoden bestimmt.

Ca. 8 Wochen nach der ersten Blutentnahme (Screeningphase) wird über einen Zeitraum von ca. 40 Wochen täglich 451 µg des Kalziumsalzes von 5-Methyl-(6S)-tetrahydrofolates oder alternativ in den jeweils ersten 21 Tagen des jeweiligen Zyklus gleichzeitig 2 oder 1.5 mg Dienogest, 15 µg Ethinylestradiol und 451 µg des Kalziumsalzes der 5-Methyl-(6S)-tetrahydrofolsäure verabreicht (Metafolin) (Tablette nach Ausführungsbeispiel 1). In einer sich daran unmittelbar anschließenden Phase wird die Gabe von 451 µg des Kalziumsalzes der 5-Methyl-(6S)-tetrahydrofolsäure in Form einer Tablette für 7 Tage fortgesetzt. Für weitere 21 Tage (zweiter Zyklus) werden erneut 2.0 oder 1-5 mg Dienogest, 15 µg Ethinylestradiol und 451 µg Metafolin und für weitere 7 Tage nur 451 µg Metafolin und so weiter, appliziert.

## Patentansprüche

1. Pharmazeutische Zusammensetzung zur Kontrazeption und zur Verminderung des Risikos angeborener Fehlbildungen, enthaltend in einer täglichen Dosis
- 2.0 mg 17α-Gyanomethyl-17-β-hydroxyestra-4,9-dien-3on (Dienogest) und 0.015 mg 17α-Ethinylestradiol (Ethinylestradiol) und (6S)-5-Methyltetrahydrofolat
oder
- 1.5 mg Dienogest und 0.015 mg Ethinylestradiol und (6S)-5-Methyltetrahydrofolat gemeinsam mit einem oder mehreren pharmazeutisch annehmbaren Hilfsstoffen/Trägern.

2. Pharmazeutische Zusammensetzung nach Anspruch 1
enthaltend ein Kalziumsalz der (6S)-5-Methyltetrahydrofolsäure in unterschiedlich geeigneten Kristallformen.

3. Pharmazeutische Zusammensetzung nach Anspruch 2
enthaltend ein kristallines oder ein mikroverkapseltes Kalziumsalz der (6S)-5-Methyltetrahydrofolsäure in unterschiedlich geeigneten Kristallformen.

4. Pharmazeutische Zusammensetzung nach Anspruch 1
enthaltend eine tägliche Dosis von 0.4 bis 1 mg (6S)-5-Methyltetrahydrofolat.

5. Pharmazeutische Zusammensetzung nach Anspruch 1
enthaltend eine tägliche Dosis von 451 µg des Kalziumsalzes der (6S)-5-Methyltetrahydrofolsäure für das (6S)-5-Methyltetrahydrofolat.

6. Kit enthaltend
- 21 tägliche Dosiseinheiten einer pharmazeutischen Zusammensetzung nach einem der Ansprüche 1 bis 4
- 7 tägliche Dosiseinheiten enthaltend (6S)-5-Methyltetrahydrofolat.

7. Kit nach Anspruch 6 enthaltend 451 µg des Kalziumsalzes der (6S)-5-Methyltetrahydrofolsäure für das 5-Methyl-(6S)-tetrahydrofolat in jeder täglichen Dosiseinheit.

8. Verwendung von
- 2.0 mg Dienogest und 0.015 mg Ethinylestradiol und (6S)-5-Methyltetrahydrofolat
oder
- 1.5 mg Dienogest und 0.015 mg Ethinylestradiol und (6S)-5-Methyltetrahydrofolat gemeinsam mit einem oder mehreren pharmazeutisch annehmbaren Hilfsstoffen/Trägern
zur Herstellung einer pharmazeutischen Zusammensetzung zur Verminderung des Risikos folatmangelbedingter angeborener Fehlbildungen in der Gravidität.

9. Verwendung von 451 µg des Kalziumsalzes der (6S)-5-Methyltetrahydrofolsäure, 2.0 oder 1.5 mg Dienogest und 0.015 mg Ethinylestradiol nach Anspruch 8.

10. Pharmazeutische Zusammensetzung, als Wirkstoffkombination 2.0 oder 1.5 mg Dienogest und 0.015 mg Ethinylestradiol und (6S)-5-Methyltetrahydrofolat enthaltend, wobei die pharmazeutische Zusammensetzung eine Tablette, vorzugsweise eine Filmtablette darstellt
mit einem Tablettenkern mit einem retardierend freizusetzenden anteiligen Gehalt zum Gesamtgehalt an Dienogest und einem Filmüberzug mit einem nicht retardierend (schnell) freizusetzenden anteiligen Gehalt zum Gesamtgehalt an Dienogest und einem nicht retardierend (schnell) freizusetzenden Gesamtgehalt an Ethinylestradiol
- und einem retardierend freizusetzenden anteiligen Gehalt zum Gesamtgehalt an (6S)-5-Methyltetrahydrofolat im Tablettenkern und einem nicht retardierend (schnell) freizusetzenden anteiligen Gehalt zum Gesamtgehalt an (6S)-5-Methyltetrahydrofolat im Filmüberzug
- oder einem retardierend freizusetzenden Gesamtgehalt an (6S)-5-Methyltetrahydrofolat im Tablettenkern
- oder einem nicht retardierend (schnell) freizusetzenden Gesamtgehalt an (6S)-5-Methyltetrahydrofolat im Filmüberzug.

11. Kit enthaltend
- 21 tägliche Dosiseinheiten einer pharmazeutischen Zusammensetzung nach Anspruch 10 und
- 7 tägliche Dosiseinheiten enthaltend (6S)-5-Methyltetrahydrofolat.

12. Kit nach Anspruch 11 enthaltend 451 µg des Kalziumsalzes der 5-Methyl-(6S)-tetrahydrofolsäure für das (6S)-5-Methyltetrahydrofolat in jeder täglichen Dosiseinheit.

13. Verwendung von
- 2.0 mg Dienogest und 0.015 mg Ethinylestradiol und (6S)-5-Methyltetrahydrofolat
oder
- 1.5 mg Dienogest und 0.015 mg Ethinylestradiol und (6S)-5-Methyltetrahydrofolat gemeinsam mit einem oder mehreren pharmazeutisch annehmbaren Hilfsstoffen/Trägern
zur Herstellung einer pharmazeutischen Zusammensetzung zur Verminderung des Risikos folatmangelbedingter angeborener Fehlbildungen nach vorangegangener längerfristiger regelmäßiger Einnahme der pharmazeutischen Zusammensetzung nach Anspruch 9 bis 11.

14. Verwendung von 451 µg des Kalziumsalzes der (6S)-5-Methyltetrahydrofolsäure, 2.0 oder 1.5 mg Dienogest und 0.015 mg Ethinylestradiol nach Anspruch 14.
